# EUROPÄISCHE PATENTSCHRIFT

(11) **EP 1 945 183 B1**
(45) Veröffentlichungstag und Bekanntmachung des Hinweises auf die Patenterteilung: **23.03.2016**
(21) Anmeldenummer: 06819138.6
(22) Anmeldetag: 25.10.2006
(51) Int. Cl.: A61K 9/14, A61K 9/20, A61K 8/81, A01N 49/00, C08L 39/00, A61K 47/32, A61Q 19/00, A61K 8/86, A61K 47/34, A61K 9/08, C08F 220/06, C08F 218/08

(54) **VERWENDUNG VON COPOLYMEREN ALS SOLUBILISATOREN FÜR IN WASSER SCHWERLÖSLICHE VERBINDUNGEN**
USE OF A COPOLYMER IN THE FORM OF A SOLUBILISER FOR A POORLY WATER-SOLUBLE COMPOUND
UTILISATION DE COPOLYMERES EN TANT QUE SOLUBILISANTS DE COMPOSES PEU SOLUBLES DANS L'EAU

(30) Priorität: 04.11.2005 DE 102005053066
(43) Veröffentlichungstag der Anmeldung: 23.07.2008
(73) Patentinhaber: BASF SE, 67056 Ludwigshafen (DE)
(72) Erfinder: BOUILLO, Nathalie, 76532 Baden-Baden (DE); PIEROBON, Marianna, 67063 Ludwigshafen (DE); WIDMAIER, Ralf, 67061 Ludwigshafen (DE); DOBRAWA, Rainer, 68167 Mannheim (DE); MEYER-BÖHM, Kathrin, 90537 Feucht (DE); LANGE, Ronald Frans Maria, 67061 Ludwigshafen (DE)
(86) Internationale Anmeldenummer: PCT/EP2006/067747
(87) Internationale Veröffentlichungsnummer: WO 2007/051743

(56) Entgegenhaltungen:
- EP-A- 0 285 038
- EP-A- 0 544 144
- EP-A- 0 953 347
- EP-A- 1 136 070
- EP-A- 1 138 322
- WO-A-01/09271
- WO-A-02/15865
- WO-A1-02/18526
- DE-A- 2 016 470
- DE-A- 3 228 384

## Beschreibung

Die vorliegende Erfindung betrifft die Verwendung von Copolymeren, die durch Polymerisation von Vinylacetat und N-Vinylcaprolactam in Gegenwart eines Polyethylenglykols erhalten werden, als Solubilisatoren für in Wasser schwerlösliche biologisch aktive Substanzen sowie die entsprechenden Zubereitungen.

Jeglicher in der Beschreibung offenbarter Gegenstand, der nicht unter die Ansprüche fällt, ist nicht Teil der vorliegenden Erfindung.

Bei der Herstellung homogener Zubereitungen von biologisch aktiven Substanzen hat die Solubilisierung von hydrophoben, also in Wasser schwerlöslichen Stoffen, eine sehr große praktische Bedeutung erlangt.

Unter Solubilisierung ist das Löslichmachen von in einem bestimmtem Lösungsmittel, insbesondere Wasser, schwer- oder unlöslichen Substanzen durch grenzflächenaktive Verbindungen, die Solubilisatoren, zu verstehen. Solche Solubilisatoren sind in der Lage, schlecht wasserlösliche oder wasserunlösliche Stoffe in klare, höchstens opaleszierende wässrige Lösungen zu überführen, ohne dass hierbei die chemische Struktur dieser Stoffe eine Veränderung erfährt (Vgl. Römpp Chemie Lexikon, 9. Auflage, Bd.5. S. 4203, Thieme Verlag, Stuttgart, 1992).

Die hergestellten Solubilisate sind dadurch gekennzeichnet, dass der schlecht wasserlösliche oder wasserunlösliche Stoff in den Molekülassoziaten der oberflächenaktiven Verbindungen, die sich in wässriger Lösung bilden, wie beispielsweise hydrophobe Domänen oder Mizellen, kolloidal gelöst vorliegt. Die resultierenden Lösungen sind stabile oder metastabile einphasige Systeme, die optisch klar bis opaleszent erscheinen.

Solubilisatoren können beispielsweise das Aussehen von kosmetischen Formulierungen sowie von Lebensmittelzubereitungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Als Solubilisatoren für pharmazeutische Arzneistoffe und kosmetische Wirkstoffe werden hauptsächlich Tenside wie ethoxyliertes Ricinusöl oder ethoxyliertes hydriertes Ricinusöl, ethoxylierte Sorbitanfettsäureester oder ethoxylierte Hydroxystearinsäure eingesetzt.

Die oben beschriebenen, bisher eingesetzten Solubilisatoren zeigen jedoch eine Reihe anwendungstechnischer Nachteile.

Die bekannten Solubilisatoren besitzen für einige schwerlösliche Arzneistoffe wie z.B. Clotrimazol nur eine geringe lösungsvermittelnde Wirkung.

In der EP-A 876 819 ist die Verwendung von Copolymeren aus mindestens 60 Gew.-% N-Vinylpyrrolidon und Amiden oder Estern mit langkettigen Alkylgruppen beschrieben.

In der EP-A 948 957 ist die Verwendung von Copolymerisaten aus monoethylenisch ungesättigten Carbonsäuren wie beispielsweise Acrylsäure und hydrophob modifizierten Comonomeren wie beispielsweise N-Alkyl- oder N,N-Dialkyl- Amiden ungesättigter Carbonsäuren mit C₈-C₃₀-Alkylresten beschrieben.

Aus der DE-A 199 350 63 sind Polyalkylenoxid-haltige Pfropfpolymere auf Basis von Vinyllactamen und Vinylacetat sowie deren Verwendung als Gashydratinhibitoren bekannt.

Aus der EP-A 953 347 ist die Verwendung von Polyalkylenoxid-haltigen Pfropfpolymerisaten als Solubilisatoren bekannt. Die dort beschriebenen Pfropfpolymerisate aus Vinylacetat und Polyalkylenoxiden stellen häufig keine Pulver sondern zähklebrige Flüssigkeiten dar, was anwendungstechnisch von Nachteil ist.

Aus der DE 20 16 470 ist bekannt, stabile wässrige Dispersionen von in Wasser schwerlöslichen optische Aufhellern herzustellen, indem die optischen Aufheller in Assoziationskomplexe überführt werden, die in wässrige Lösungen von Polymeren einträgt. Als Polymere werden beispielsweise Pfropfpolymere aus N-Vinylpyrrolidon, und/oder Vinylacetat und Polyethern genannt.

Aus der EP-A 0 285 038 ist die Verwendung von Pfropfpolymeren, die durch Pfropfen von Polyalkylenoxiden mit N-Vinylpyrrolidon und Vinylestern wie Vinylacetat erhalten werden, als Vergraungsinhibitoren beim Waschen von Textilgütern.

Aus der WO 02/15865 ist die Verwendung von Polymerisaten, die erhalten werden durch radikalische Polymerisation von Vinylestern wie Vinylacetat in Gegenwart von Polyethern, für hautkosmetische Formulierungen und in der dekorativen Kosmetik bekannt.

In der EP-A 11 36 070 ist die Verwendung von Polymerisaten, erhalten durch radikalische Polymerisation von Vinylestern in Gegenwart von Polyethern, für die Herstellung von Weichkapseln bekannt.

In der EP-A 11 38 322 ist die Verwendung von Polymerisaten, erhalten durch radikalische Polymerisation von Vinylestern in Gegenwart von Polyethern, für die Herstellung von Hartkapseln bekannt.

Aus der EP-A 0 544 144 sind feste pharmazeutische Retardformen bekannt, in der der Wirkstoff durch Schmelzextrusion in eine die Freisetzung verlangsamende Polymermatrix eingebettet wird. Die Polymermatrix enthält mindestens ein wasserunlösliches Poly(meth)acrylatpolymer und als weitere Polymerkomponente eine wasserlösliche Hydroxyalkylcellulose und/oder ein N-Vinylpyryrrolidonpolymerisat mit bis zu 50 Gew.-% Vinylacetat.

Aus der DE-A 32 28 384 ist ein Verfahren zu Verbesserung des schwerlöslichen Wirkstoffs Glibenclamid bekannt, welches dadurch gekennzeichnet ist, dass der Wirkstoff unter Verwendung weiterer Stoffe in eine amorphe Form überführt wird. Als weitere stoffe werden Zuckeralkohole, Polyvinylpyrrolidone, Copolymerisate von Vinylpyrrolidon/Vinylacetat, kolloidale Kieselsäuren, Cellulosen sowie Cellulosederivate genannt. Diese weitern Stoffe sollen die Rekristallisation des Wirkstoffs verhindern.

Eine weitere wünschenswerte Anforderung an Solubilisatoren ist die Fähigkeit, mit schwerlöslichen Substanzen sogenannte "feste Lösungen" auszubilden. Der Begriff "feste Lösung" bezeichnet einen Zustand, in dem eine Substanz mikrodispers oder im Idealfall molekulardispers in einer festen Matrix, beispielsweise einer Polymermatrix, verteilt ist. Solche festen Lösungen führen zum Beispiel bei Verwendung in festen pharmazeutischen Darreichungsformen eines schwerlöslichen Wirkstoffs zu einer verbesserten Freisetzung des Wirkstoffs. Eine wichtige Anforderung an solche feste Lösungen ist, dass sie auch bei Lagerung über längere Zeit stabil sind, d.h., dass der Wirkstoff nicht auskristallisiert. Weiterhin ist auch die Kapazität der festen Lösung, mit anderen Worten die Fähigkeit der Ausbildung von stabilen festen Lösungen mit möglichst hohen Wirkstoffgehalten, von Bedeutung.

Bei der Ausbildung von festen Lösungen spielt neben der grundsätzlichen Fähigkeit der Solublisatoren zur Bildung von festen Lösungen auch die Hygroskopizität der Solubilisatoren eine bedeutende Rolle. Solubilisatoren, die aus der Umgebungsluft zuviel Wasser aufnehmen, führen zu einem Zerfliessen der festen Lösung und der unerwünschten Kristallisation der Wirkstoffe. Auch bei der Verarbeitung zu Darreichungsformen kann eine zu große Hygroskopizität Probleme bereiten.

Die bisher bekannten polymeren Solubilisatoren weisen die Nachteile auf, dass sie keine stabilen festen Lösungen ausbilden. Ausserdem lassen sie noch Raum für Verbesserungen, was die Solubilisierung in wässrigen Systemen betrifft. Auch hinsichtlich der Verarbeitbarkeit weisen einige der bekannten Solubilisatoren aufgrund ihrer Neigung zu Klebrigkeit Nachteile auf, da sie keine ausreichend fließfähigen Pulver darstellen.

Es bestand daher die Aufgabe, neue und verbesserte Solubilisatoren für pharmazeutische, kosmetische, lebensmitteltechnische, agrotechnische oder sonstige technische Anwendungen bereitzustellen, die die geschilderten Nachteile nicht aufweisen.

Die Aufgabe wurde erfindungsgemäß gelöst durch die Verwendung von wasserlöslichen oder wasserdispergierbaren Copolymerisaten, die erhalten werden durch radikalisch initiierte Polymerisation einer Mischung aus
i) 30 bis 80 Gew.-% N-Vinylcaprolactam,
ii) 10 bis 50 Gew.-% Vinylacetat und
iii) 10 bis 50 Gew.-% eines Polyethylenglykols,
mit der Maßgabe, dass die Summe von i), ii) und iii) gleich 100 Gew.-% ist,

Gemäß einer Ausführungsform der Erfindung werden bevorzugte Polymerisate erhalten aus:
i) 30 bis 70 Gew.-% N-Vinylcaprolactam,
ii) 15 bis 35 Gew.-% Vinylacetat, und
iii) 15 bis 35 Gew.-% eines Polyethylenglykols,
und besonders bevorzugte Polymerisate aus:
i) 40 bis 60 Gew.-% N-Vinylcaprolactam
ii) 15 bis 35 Gew.-% Vinylacetat
iii) 15 bis 30 Gew.-% eines Polyethylenglykols.

Gemäß einer weiteren Ausführungsform der Erfindung enthalten bevorzugte Polymerisate 10 bis 35 Gew.-% eines Polyethers.

Insbesondere bevorzugt sind Polymerisate aus
i) 40 bis 60 Gew.-% N-Vinylcaprolactam
ii) 15 bis 35 Gew.-% Vinylacetat
iii) 10 bis 30 Gew.-% eines Polyethylenglykols.

Auch für die bevorzugten und besonders bevorzugten Zusammensetzungen gilt die Maßgabe, dass die Summe der Komponeten i), ii), und iii) gleich 100 Gew.-% beträgt.

Die Polyalkylenglykole können Molekulargewichte von 1000 bis 100000 D [Dalton], vorzugsweise 1500 bis 35000 D, besonders bevorzugt 1500 bis 10000 D, aufweisen. Die Molekulargewichte werden ausgehend von der gemäß DIN 53240 gemessenen OH-Zahl bestimmt.

Allgemeine Verfahren zur Herstellung der erfindungsgemäßen Copolymerisate sind an sich bekannt. Die Herstellung erfolgt durch radikalisch initiierte Polymerisation, bevorzugt Lösungspolymerisation, in nichtwässrigen, organischen Lösungsmitteln oder in gemischt nichtwässrigen/wässrigen Lösungsmitteln.

Geeignete nichtwässrige organische Lösungsmittel sind beispielsweise Alkohole, wie Methanol, Ethanol, n-Propanol, und Isopropanol sowie Glykole, wie Ethylenglykol und Glycerin.

Weiterhin eignen sich als Lösungsmittel Ester wie beispielsweise Ethylacetat, n-Propylacetat, Isopropylacetat, Isobutylacetat oder Butylacetat.

Die Polymerisation wird vorzugsweise bei Temperaturen von 60 bis 100°C durchgeführt.

Zur Initiierung der Polymerisation werden radikalische Initiatoren eingesetzt. Die verwendeten Mengen an Initiator bzw. Initiatorgemischen bezogen auf eingesetztes Monomer liegen zwischen 0,01 und 10 Gew.-%, vorzugsweise zwischen 0,3 und 5 Gew.-%.

Je nach Art des verwendeten Lösungsmittels eignen sich sowohl organische als auch anorganische Peroxide wie Natriumpersulfat oder Azostarter wie Azo-bis-isobutyronitril, Azo-bis-(2-amidopropan)dihydrochlorid oder 2,2'-Azo-bis-(2-methyl-butyronitril).

Peroxidische Initiatoren sind beispielsweise Dibenzoylperoxid, Diacetylperoxid, Succinylperoxid, tert.-Butylperpivalat, tert.-Butylperethylhexanoat, tert.-Butylperneodecanoat, tert.-Butylpermaleinat, Bis-(tert.-Butylper)-cyclohexan, tert.-Butylper-isopropylcarbonat, tert.-Butylperacetat, 2,2-Bis-(tert.-butylper)-butan, Dicumylperoxid, Di-tert.-amylperoxid, Di-tert.-butylperoxid, p-Menthanhydroperoxid, Pinanhydroperoxid, Cumolhydroperoxid, tert.-Butylhydroperoxid, Wasserstoffperoxid sowie Mischungen der genannten Initiatoren. Die genannten Initiatoren können auch in Kombination mit Redoxkomponenten wie Ascorbinsäure verwendet werden.

Besonders geeignet als Initiatoren sind tert.-Butylperneodecanoat, tert.-Butylperpivalat oder tert.-Butylperethyl-hexanoat.

Die radikalische Polymerisation kann gegebenenfalls in Gegenwart von Emulgatoren, gegebenenfalls weiteren Schutzkolloiden, gegebenenfalls Molekulargewichtsreglern, gegebenenfalls Puffersystemen und gegebenenfalls nachfolgender pH-Einstellung mittels Basen oder Säuren statt.

Als Molekulargewichtsregler eignen sich Schwefelwasserstoffverbindungen wie Alkylmercaptane, z.B. n-Dodecylmercaptan, tert.-Dodecylmercaptan, Thioglykolsäure und deren Ester, Mercaptoalkanole wie Mercaptoethanol. Weitere geeignete Regler sind z.B. in der DE 197 12 247 A1, Seite 4, genannt. Die erforderliche Menge der Molekulargewichtsregler liegt im Bereich von 0 bis 5 Gew.-% bezogen auf die zu polymerisierenden (Co-)Monomerenmenge. Im falle dass Regler verwendet werden, liegt die eingesetzte Menge insbesondere im Bereich von 0,05 bis 2 Gew.-%, besonders bevorzugt 0,1 bis 1,5 Gew.-%. Ganz besonders bevorzugt ist jedoch die Polymerisation in Abwesenheit eines Reglers.

Gegebenenfalls können auch Emulgatoren verwendet werden, beispielsweise ionische oder nichtionische Tenside, deren HLB-Wert üblicherweise im Bereich von 3 bis 13 liegt. Zur Definition des HLB-Werts wird auf die Veröffentlichung von W.C. Griffin, J. Soc. Cosmetic Chem., Band 5, 249 (1954) hingewiesen. Die Menge an Tensiden bezogen auf das Polymerisat kann 0 bis 10 Gew.-%, bevorzugt 0 bis 5 Gew.-%, betragen.

Das Monomere bzw. die Monomerenmischung oder die Monomer(en)emulsion werden zusammen mit dem Initiator, der i.a. in Lösung vorliegt, in einem Rührreaktor bei der Polymerisationstemperatur vorlegt (Batch-Prozess), oder gegebenenfalls kontinuierlich oder in mehreren aufeinanderfolgenden Stufen in den Polymerisationsreaktor zudosiert (Zulaufverfahren). Beim Zulaufverfahren ist es üblich, dass der Reaktor vor Beginn der eigentlichen Polymerisation neben dem Lösungsmittel (um eine Rührung des Reaktionsgemisches zu ermöglichen) bereits mit Teilmengen, selten der gesamten für die Polymerisation vorgesehenen Menge, der Einsatzstoffe wie Emulgatoren, Schutzkolloiden, Monomeren, Regler usw. oder Teilmengen der Zuläufe (i.a. Monomer- oder Emulsionszulauf sowie Initiatorzulauf) befüllt wird.

Die Polymersation kann sowohl bei Normaldruck als auch in geschlossenem Reaktor bei erhöhtem Druck durchgeführt werden. Dabei kann entweder bei dem Druck polymerisiert werden, der sich während der Reaktion einstellt, oder der Druck kann durch Aufpressen eines Gases oder Evakuieren eingestellt werden. Weiterhin kann der Druck auch durch zeitweises Entspannen des Reaktors in den Kühler kontrolliert werden.

Ein für die Polymerisation verwendetes nicht-wässriges Lösungsmittel kann anschließend mittels Wasserdampfdestillation entfernt und gegen Wasser ausgetauscht werden. Dabei wird üblicherweise zunächst das nicht-wässrige Lösungsmittel soweit wie möglich rein abdestilliert und anschließend durch Einleiten von Wasserdampf komplett gegen Wasser ausgetauscht.

Nach der Polymerisation können allgemein bekannte Verfahren zur Restmonomerabsenkung eingesetzt werden. Solche Verfahren sind beispielsweise die weitere Zugabe von Initiator am Ende der Polymerisation, die Hydrolyse von Vinyllactam-Monomeren durch Zugabe von Säuren, Behandeln der Polymerlösung mit festen Phasen wie Ionenaustauschern, Zulauf eines gut copolymerisierenden Monomers, Membranfiltration und weitere übliche Methoden.

Der Feststoffgehalt der erhaltenen wässrigen Polymerisat-Dispersionen oder Lösungen beträgt in der Regel 10 bis 70 Gew.-%, bevorzugt 15 bis 60 Gew.-%, besonders bevorzugt 15 bis 40 Gew.-%.

Die Polymerisat-Dispersionen oder Lösungen können durch verschiedene Trocknungsverfahren wie z.B. Sprühtrocknung, Fluidized Spray Drying, Walzentrocknung, Schaufeltrocknung, Bandtrocknung oder Gefriertrocknung in Pulverform oder in Granulate überführt werden. Es kann sich auch empfehlen, bei der Sprühtrocknung Additive wie beispielweise hochdisperse Kieselsäure oder hydrophob modifizierte hochdisperse Kieselsäure zuzusetzen.

Die Copolymerisate werden als wässrige Dispersionen oder wässrige Lösungen oder nach Entfernung des Wasseranteils als gut riesel- und fließfähige wasserdispergierbare oder wasserlösliche Pulver gewonnen.

Die Polymerisate weisen K-Werte nach Fikentscher im Bereich von 10 bis 60, vorzugsweise 15 bis 40, gemessen in einer 1 gew.-%igen ethanolischen Lösung, auf.

### Anwendungen:

Die erfindungsgemäß zu verwendenden Copolymere lassen sich grundsätzlich auf allen Gebieten einsetzen, bei denen in Wasser nur schwerlösliche oder unlösliche Substanzen entweder in wässrigen Zubereitungen zum Einsatz kommen sollen oder ihre Wirkung in wässrigem Milieu entfalten sollen. Die Copolymere finden demgemäß Verwendung als Solubilisatoren von in Wasser schwerlöslichen Substanzen, insbesondere biologisch aktiven Substanzen.

Der Begriff "in Wasser schwerlöslich" umfasst erfindungsgemäß auch praktisch unlösliche Substanzen und bedeutet, dass für eine Lösung der Substanz in Wasser bei 20 °C mindestens 30 bis 100 g Wasser pro g Substanz benötigt wird. Bei praktisch unlöslichen Substanzen werden mindestens 10.000 g Wasser pro g Substanz benötigt.

Im Sinne der vorliegenden Erfindung sind unter in Wasser schwerlösliche biologisch aktive Substanzen pharmazeutische Wirkstoffe für Mensch und Tier, kosmetische oder agrochemische Wirkstoffe oder Nahrungsergänzungsmittel oder diätetische Wirkstoffe zu verstehen.

Weiterhin kommen als zu solubilisierende schwerlösliche Substanzen auch Farbstoffe wie anorganische oder organische Pigmente in Betracht.

Durch die vorliegende Erfindung werden insbesondere amphiphile Verbindungen für die Anwendung als Lösungsvermittler für pharmazeutische und kosmetische Zubereitungen sowie für Lebensmittelzubereitungen zur Verfügung gestellt. Sie besitzen die Eigenschaft, schwer lösliche Wirkstoffe auf dem Gebiet der Pharmazie und Kosmetik, schwerlösliche Nahrungsergänzungsmittel, beispielsweise Vitamine und Carotinoide aber auch schwerlösliche Wirkstoffe für den Einsatz in Pflanzenschutzmitteln sowie veterinärmedizinische Wirkstoffe zu solubilisieren.

### Solubilisatoren für Kosmetik:

Erfindungsgemäßen können die Copolymere als Solubilisatoren in kosmetischen Formulierungen eingesetzt werden. Beispielsweise eignen sie sich als Solubilisatoren für kosmetische Öle. Sie besitzen ein gutes Solubilisiervermögen für Fette und Öle wie Erdnussöl, Jojobaöl, Kokosnussöl, Mandelöl, Olivenöl, Palmöl, Ricinusöl, Sojaöl oder Weizenkeimöl oder für etherische Öle wie Latschenkiefernöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Salbeiöl, Wacholderöl, Zitronenöl, Anisöl, Kardamonöl; Pfefferminzöl, Campheröl etc. oder für Mischungen aus diesen Ölen.

Weiterhin können die erfindungsgemäßen Polymere als Solubilisatoren für in Wasser schwerlösliche oder unlösliche UV-Absorber wie beispielsweise 2-Hydroxy-4-methoxybenzophenon (Uvinul® M 40, Fa. BASF), 2,2',4,4'-Tetrahydroxybenzophenon (Uvinul® D 50), 2,2'-Dihydroxy-4,4'-dimethoxybenzophenon (Uvinul®D49), 2,4-Dihydroxybenzophenon (Uvinul® 400), 2-Cyano-3,3-diphenylacrylsäure-2'-ethylhexylester (Uvinul® N 539), 2,4,6-Trianilino-p-(carbo-2'-ethylhexyl-1'-oxi)-1,3,5-triazin (Uvinul^{®} T 150), 3-(4-Methoxybenzyliden)-campher (Eusolex^{®} 6300, Fa. Merck), N,N-Dimethyl-4-aminobenzoesäure-2-ethylhexylester (Eusolex® 6007), Salicylsäure-3,3,5-trimethylcyclohexylester, 4-Isopropyl-dibenzoylmethan (Eusolex® 8020), p-Methoxyzimtsäure-2-ethylhexylester und p-Methoxyzimtsäure-2-isoamylester sowie Mischungen davon verwendet werden.

Gegenstand der vorliegenden Erfindung sind daher auch kosmetische Zubereitungen, die mindestens eines der erfindungsgemäßen Copolymere der eingangs genannten Zusammensetzung als Solubilisatoren enthalten. Bevorzugt sind solche Zubereitungen, die neben dem Solubilisator einen oder mehrere schwerlösliche kosmetische Wirkstoffe, beispielsweise die oben genannten Öle oder UV-Absorber enthalten.

Bei diesen Formulierungen handelt es sich um Solubilisate auf Wasser oder Wasser/Alkohol Basis. Die erfindungsgemäßen Solubilisatoren werden im Verhältnis von 0,2:1 bis 20:1, bevorzugt 1:1 bis 15:1, besonders bevorzugt 2:1 bis 12:1 zum schwerlöslichen kosmetischen Wirkstoff eingesetzt.

Der Gehalt an erfindungsgemäßem Solubilisator in der kosmetischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

Zusätzlich können dieser Formulierung weitere Hilfsstoffe zugesetzt werden, beispielsweise nichtionische, kationische oder anionische Tenside wie Alkylpolyglycoside, Fettalkoholsulfate, Fettalkoholethersulfate, Alkansulfonate, Fettalkoholethoxilate, Fettalkoholphosphate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester, Fettsäureamide, Fettsäurealkanolamide, quartäre Ammoniumverbindungen, Alkylphenoloxethylate, Fettaminoxethylate, Cosolvenzien wie Ethylenglykol, Propylenglykol, Glycerin u.a..

Als weitere Bestandteile können natürliche oder synthetische Verbindungen, z.B. Lanolinderivate, Cholesterinderivate, Isopropylmyristat, Isopropylpalmitat, Elektrolyte, Farbstoffe, Konservierungsmittel, Säuren (z.B. Milchsäure, Zitronensäure) zugesetzt werden.

Diese Formulierungen finden beispielsweise in Badezusatzpräparaten wie Badeölen, Rasierwässern, Gesichtswässern, Haarwässern, Eau de Cologne, Eau de Toilette sowie in Sonnenschutzmitteln Verwendung. Ein weiteres Einsatzgebiet ist der Bereich Oral Care, beispielsweise in Mundwässern, Zahnpasten, Haftcremes für Zahnprothesen und dergleichen.

Beschreibung der Solubilisierungsmethode:
Bei der Herstellung der Solubilisate für kosmetische Formulierugen können die erfindungsgemäßen Copolymere als 100%ige Substanz oder bevorzugt als wässrige Lösung eingesetzt werden.

Üblicherweise wird der Solubilisator in Wasser gelöst und mit dem jeweils zu verwendenden schwerlöslichen kosmetischen Wirkstoff intensiv vermischt.

Es kann aber auch der Solubilisator mit dem jeweils zu verwendenden schwerlöslichen kosmetischen Wirkstoff intensiv vermischt werden und anschließend unter ständigem Rühren mit demineralisiertem Wasser versetzt werden.

### Solubilisatoren für pharmazeutische Anwendungen:

Die beanspruchten Copolymerisate eignen sich ebenso für die Verwendung als Solubilisator in pharmazeutischen Zubereitungen jeder Art, die dadurch gekennzeichnet sind, dass sie einen oder mehrere in Wasser schwer lösliche oder wasserunlösliche Arzneistoffe sowie Vitamine und/oder Carotinoide enthalten können. Insbesondere handelt es sich dabei um wässrige Lösungen bzw. Solubilisate zur oralen Applikation.
So eignen sich die beanspruchten Copolymere zum Einsatz in oralen Darreichungsformen wie Tabletten, Kapseln, Pulvern, Lösungen. Hier können Sie den schwerlöslichen Arzneistoff mit einer erhöhten Bioverfügbarkeit zur Verfügung stellen. Insbesondere finden feste Lösungen aus Wirkstoff und Solubilisator Verwendung.

Bei der parenteralen Applikation können neben Solubilisaten auch Emulsionen, beispielsweise Fettemulsionen eingesetzt werden. Auch für diesen Zweck eignen sich die beanspruchten Copolymere um einen schwerlöslichen Arzneistoff zu verarbeiten.

Pharmazeutische Formulierungen der oben genannten Art können durch Verarbeiten der beanspruchten Copolymere mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die verwendeten pharmazeutischen Wirkstoffe sind in Wasser unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Als Beispiele seien hier Benzodiazepine, Antihypertensiva, Vitamine, Cytostatika - insbesondere Taxol, Anästhetika, Neuroleptika, Antidepressiva, antiviral wirksame Mittel wie beispielsweise Anti-HIV wirksame Mittel, Antibiotika, Antimykotika, Antidementiva, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, , Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Eine mögliche Herstellvariante ist das Auflösen des Solubilisators in der wäßrigen Phase, gegebenenfalls unter leichtem Erwärmen und das anschließende Lösen des Wirkstoffs in der wässrigen Solubilisatorlösung. Das gleichzeitige Auflösen von Solubilisator und Wirkstoff in der wässrigen Phase ist ebenfalls möglich.

Die Verwendung der erfindungsgemäßen Copolymere als Solubilisator kann beispielsweise auch in der Weise erfolgen, dass der Wirkstoff in dem Solubilisator, gegebenenfalls unter Erwärmen, dispergiert wird und unter Rühren mit Wasser vermischt wird.

Weiterhin können die Solubilisatoren auch in der Schmelze mit den Wirkstoffen verarbeitet werden. Insbesondere können auf diese Weise feste Lösungen erhalten werden. Hierfür eignet sich unter anderem auch das Verfahren der Schmelzextrusion. Eine weitere Möglichkeit zur Herstellung von festen Lösungen ist auch, Lösungen von Solubilisator und Wirkstoff in geeigneten organischen Lösungsmitteln herzustellen und das Lösungsmittel anschliessend durch übliche Verfahren zu entfernen.

Gegenstand der Erfindung sind daher auch allgemein pharmazeutische Zubereitungen, die mindestens eines der erfindungsgemäßen Copolymere als Solubilisator enthalten. Bevorzugt sind solche Zubereitungen, die neben dem Solubilisator einen in Wasser schwerlöslichen oder wasserunlöslichen pharmazeutischen Wirkstoff, beispielsweise aus den oben genannten Indikationsgebieten enthalten.

Besonders bevorzugt sind von den oben genannten pharmazeutischen Zubereitungen solche, bei denen es sich um oral applizierbare Formulierungen handelt.

Der Gehalt an erfindungsgemäßem Solubilisator in der pharmazeutischen Zubereitung liegt, abhängig vom Wirkstoff, im Bereich von 1 bis 75 Gew.-%, bevorzugt 5 bis 60 Gew.-%, besonders bevorzugt 5 bis 50 Gew.-%.

Eine weitere besonders bevorzugte Ausführungsform bezieht sich auf pharmazeutische Zubereitungen, bei denen die Wirkstoffe und der Solubilisator als feste Lösung vorliegen. Hierbei beträgt das Gewichtsverhältnis von Solubilisator zu Wirkstoff vorzugsweise von 1:1 bis 4:1, kann jedoch bis100:1, insbesondere bis 15:1 betragen. Es kommt nur darauf an, dass bei Einsatz in der fertigen Arzneiform zum einen eine wirksame Menge Wirkstoff in der Arzneiform enthalten ist, und zum anderen bei oralen Arzneiformen die Formen nicht zu gross werden.
Solubilisatoren für Lebensmittelzubereitungen:
Neben der Anwendung in der Kosmetik und Pharmazie eignen sich die erfindungsgemäßen Copolymeren auch als Solubilisatoren im Lebensmittelbereich für schwer wasserlösliche oder wasserunlösliche Nähr-, Hilfs- oder Zusatzstoffe, wie z.B. fettlösliche Vitamine oder Carotinoide. Als Beispiele seien mit Carotinoiden gefärbte Getränke genannt.

Solubilisatoren für Pflanzenschutzzubereitungen:
Die Anwendung der erfindungsgemäßen Copolymere als Solubilisatoren in der Agrochemie kann u.a. Formulierungen umfassen, die Pestizide, Herbizide, Fungizide oder Insektizide enthalten, vor allem auch solche Zubereitungen von Pflanzenschutzmitteln, die als Spritz- oder Gießbrühen zum Einsatz kommen.

Die erfindungsgemäßen Copolymere zeichnen sich durch eine besonders gute solubilisierende Wirkung aus. Sie sind auch in der Lage, sogenannte feste Lösungen mit schwerlöslichen Substanzen auszubilden. Als feste Lösungen werden erfindungsgemäß Systeme bezeichnet, in denen bei visueller Begutachtung keine kristallinen Anteile der schwerlöslichen Substanz zu erkennen sind. Weiterhin sind bei visueller Begutachtung der stabilen festen Lösungen auch keine amorphen Bestandteile zu erkennen. Die visuelle Begutachtung erfolgt mit einem Lichtmikroskop bei 40facher Vergrößerung.

In den folgenden Beispielen wird die Herstellung und Verwendung der erfindungsgemäßen Copolymere näher erläutert.

### Herstellung der Copolymerisate

Verwendete Abkürzungen:
VCap: N-Vinylcaprolactam
VP: N-Vinylpyrrolidon
VAc: Vinylacetat
PEG: Polyethylenglykol

### Beispiel 1

Vorlage:
   165,0 g Ethylacetat,
   100,0 g PEG 6000,
   20,0 g Vinylacetat,
   10,50 g von Zulauf 2
Zulauf 1:
   500 g Vinylcaprolactam
   180 g Vinylacetat
   100 g Ethylacetat
Zulauf 2:
   10,50 g tert-Butylperethylhexanoat (98 gew.%-ig)
   94,50 g Ethylacetat

In einer Rührapparatur wurde die Vorlage ohne die Teilmenge von Zulauf 2 unter einer N₂-Atmosphäre auf 77°C aufgeheizt. Wenn die Innentemperatur von 77°C erreicht war, wurde die Teilmenge von Zulauf 2 zugegeben und 15 min anpolymerisiert. Anschließend wurden Zulauf 1 in 5h und Zulauf 2 in 2 h zudosiert. Nachdem alle Zuläufe zudosiert waren, wurde das Reaktionsgemisch noch 3h nachpolymerisiert. Nach der Nachpolymerisation wurde das Reaktionsgemisch mit 500 ml Wasser verdünnt. Flüchtige Bestandteile wurden durch Wasserdampfdestillation entfernt. Die wässrige Lösung wurde gefriergetrocknet. Die Copolymeren wurden nach Mahlung als sehr gut fließfähige Pulver erhalten.

Die weiteren Copolymere gemäß den Beispiele 2-5 wurden analog mit leicht veränderter Zusammensetzung hergestellt.

### Beispiel 2

Vorlage:
   165 g Ethylacetat
   100,0 g PEG 6000
   22,0 g Vinylacetat
   10,50 g von Zulauf 2
Zulauf 1:
   480 g Vinylcaprolactam
   198 g Vinylacetat
   100 g Ethylacetat
Zulauf 2:
   10,50 g tert-Butylperethylhexanoat (98 gew.%-ig)
   94,50 g Ethylacetat

### Beispiel 3

Vorlage:
   25 g Ethylacetat
   104,0 g PEG 6000,
   1,0 g von Zulauf 2
Zulauf 1: 240 g Vinylacetat
Zulauf 2:
   456 g Vinylcaprolactam
   240 g Ethylacetat
Zulauf 3:
   10,44 g tert-Butylperpivalat (75 gew.%-ig in Aliphatengemisch)
   67,90 g Ethylacetat

### Beispiel 4

Vorlage:
   25 g Ethylacetat
   112,0 g PEG 6000,
   1,0 g von Zulauf 2
Zulauf 1:
   408 g Vinylcaprolactam
   280 g Vinylacetat
   240 g Ethylacetat
Zulauf 2: 10,32 g tert-Butylperpivalat(75 gew.%-ig in Aliphatengemisch) 67,10 g Ethylacetat

### Beispiel 5

Vorlage:
25 g Ethylacetat,
112,0 g PEG 6000,
1,0 g von Zulauf 2

Zulauf 1:
428,0 g Vinylcaprolactam
260,0 g Vinylacetat
240 g Ethylacetat

Zulauf 2: 10,32 g tert-Butylperpivalat(75 gew.%-ig in Aliphatengemisch) 67,10 g Ethylacetat

| Beispiel Nr. | Zusammensetzung in Gew.-% | | | K-Wert (1gew.-%ig in Ethanol) |
|---|---|---|---|---|
| | PEG 6000 | VCap | VAc | |
| 1 | 12,5 | 62,5 | 25 | 18,5 |
| 2 | 12,5 | 60 | 27,5 | 40,4 |
| 3 | 13 | 57 | 30 | 19,8 |
| 4 | 14 | 51 | 35 | 25,2 |
| 5 | 14 | 53,5 | 32,5 | 22,4 |

### Beispiel 6

Vorlage: 50 g Butylacetat, 150,0 g PEG 6000, 1,0 g von Zulauf 3
Zulauf 1: 500 g VCap, 120,0 g Butylacetat
Zulauf 2: 350,0 g VAc, 80, 0 g Butylacetat
Zulauf 3: 12,75 g tert-Butylperpivalat (75 gew.%-ig in Aliphatengemisch), 117,25 g Butylacetat

In einer Rührapparatur wurde die Vorlage unter einer N₂-Atmosphere auf 77°C aufgeheizt. Wenn die Temperatur erreicht war, wurden Zulauf 1, Zulauf 2 und Zulauf 3 gestartet. Zulauf 1 wurde in 5h, Zulauf 2 in 2 h zudosiert und Zulauf 3 in 5,5 h zudosiert. Nachdem alle Zuläufe zudosiert waren, wurde das Reaktionsgemisch noch 4h nachpolymerisiert. Nach der Nachpolymerisation wurde das Reaktionsgemisch mit 500 ml Lösungsmittel verdünnt. Flüchtige Bestandteile wurden durch Wasserdampfdestillation entfernt. Die wässrige Lösung wurde gefriergetrocknet. Die Copolymeren wurden nach Mahlung als sehr gut fließfähige Pulver erhalten.

Analog wurden die Copolymeren gemäß den Beispielen 7 bis 17 hergestellt.

| Beispiel Nr. | Zusammensetzung in Gew.-% | | | K-Wert (1gew.-%ig in Ethanol) |
|---|---|---|---|---|
| | PEG 6000 | VCap | VAc | |
| 6 | 15 | 50 | 35 | 32,2 |
| 7 | 20 | 50 | 30 | 21,7 |
| 8 | 25 | 50 | 25 | 22,7 |
| 9 | 30 | 50 | 20 | 23,6 |
| 10 | 35 | 50 | 15 | 25,1 |
| 11 | 50 | 40 | 10 | 24,3 |

| Beispiel Nr. | Zusammensetzung in Gew.-% | | | K-Wert (1gew.-%ig in Ethanol) |
|---|---|---|---|---|
| | PEG 1500 | VCap | VAc | |
| 12 | 15 | 50 | 35 | 32,2 |
| 13 | 25 | 50 | 25 | 23,1 |
| 14 | 35 | 50 | 15 | 29,7 |

| Beispiel Nr. | Zusammensetzung in Gew.-% | | | K-Wert (1gew.-%ig in Ethanol) |
|---|---|---|---|---|
| 15 | PEG 9000 15 | VCap 50 | VAc 35 | 30,5 |
| 16 | PEG 1500 20 | VP 60 | VAc 20 | 35,6 |
| 17 | PEG 6000 20 | VP 60 | VAc 20 | 35,8 |

### Beispiel 18

Vorlage: 40 g Ethylacetat, 120,0 g PEG 6000, 1,28 g von Zulauf 2
Zulauf 1: 400 g VCap, 280,0 g Vinylacetat, 225,0 g Ethylacetat
Zulauf 2: 10,2 g tert-Butylperpivalat (75 gew.%-ig in Aliphatengemisch), 118,4 g Ethylacetat

In einer Rührapparatur wurde die Vorlage unter einer N₂-Atmosphere auf 77°C aufgeheizt. Wenn die Temperatur erreicht war, wurden die Zuläufe gestartet. Zulauf 1 wurde in 5h, Zulauf 2 in 5,5 h zudosiert. Nachdem alle Zuläufe zudosiert waren, wurde das Reaktionsgemisch noch 3h nachpolymerisiert. Nach der Nachpolymerisation wurde das Reaktionsgemisch mit ca. 500 ml Lösungsmittel verdünnt. Flüchtige Bestandteile wurden durch Wasserdampfdestillation entfernt. Die wässrige Lösung wurde gefriergetrocknet. Die Copolymeren wurden nach Mahlung als sehr gut fließfähige Pulver erhalten.

### Beispiel 19

Analog Beispiel 18, jedoch mit tert-Butylperethylhexanoat bei einer Badtemperatur von 85°C.

### Beispiel 20

Analog Beispiel 19, jedoch mit folgenden Zuläufen:
Vorlage: 120,0 g PEG 6000
Zulauf 1: 160 g VCap, 280,0 g Vinylacetat, 50,0 g Ethylacetat
Zulauf 2: 10,2 g tert-Butylperpivalat (75 gew.%-ig in Aliphatengemisch), 91,80 g Ethylacetat
Zulauf 3: 240 g VCap, 242,0 g Ethylacetat

Zulauf 1 wurde in 2h, Zulauf 2 in 5.5 h zugeben. Sofort nach Ende von Zulauf 1 wurde Zulauf 3 gestartet.

### Beispiel 21

Analog Beispiel 19, Monomerenzusammensetzung in Gew.-%: 15 PEG/ 55 VCap/ 30 Vac.

### Beispiel 22

Analog Beispiel 19, mit tert-Butanol als Lösungsmittel.

### Beispiel 23

Analog Beispiel 18, nach Ende der Nachpolymerisation Zugabe von 9,07 g tert-Butylperpivalat in 81,6 g Ethylacetat und anschließende Nachpolymerisation für 2h zur Restmonomerabsenkung.

### Beispiel 24

Analog Beispiel 21, aber mit Monomerenzusammensetzung in Gew.-%: 15 PEG/ 50 VCap/ 35 VAc, verdoppelten Mengen und in einer Druckapparatur bei 90°C. Dabei stellte sich ein Druck im Bereich von 0.2 MPa ein.

### Beispiel 25

### Sprühtrocknung

Ein Polymer, das analog Bsp. 18 hergestellt wurde, wurde sprühgetrocknet. Die Trocknung verlief störungsfrei.
- Zerstäuber:: Zweistoffdüse, 1,3 mm Teflon
- Additiv:: keines
- Feststoffgehalt:: 15 Gew%
- Eingangstemp.:: 121°C
- Ausgangstemp.:: 55°C
- Ausbeute:: 77%
- Farbe:: weiß
- Pulvereigenschaft:: leicht verblockt

Der Zusatz von hochdisperser Kieselsäure als Sprühhilfsmittel (Additiv) verbesserte die bereits guten Eigenschaften und ergabt ein frei-fließendes Pulver.

### Beispiel 26

Eine gemäß Beispiel 1 erhaltene wässrige Polymerlösung wurde analog Beispeil 25 durch Sprühtrocknung aufgearbeitet.

### Vergleichsbeispiele

Zum Vergleich wurden wie in der EP-A 953 437 (Beispiele 1, 3; Tabelle1) beschrieben Pfropfpolymerisate der folgenden Zusammensetzung hergestellt: Vergleichsbeispiel A: 70 Gew.-% VAc, 30 Gew.-% PEG 6000 Vergleichsbeispiel B: 70 Gew.-% VAc, 30 Gew.-% PEG 1500 Diese Polymerisate wiesen eine auffallende Klebrigkeit auf. Herstellung von festen Lösungen: Allgemeine Vorschrift

Zur Herstellung des Polymer-Wirkstoff-Gemischs wurden der Wirkstoff und das Polymer im Gewichtsverhältnis 1:1 in ein geeignetes Glasgefäß eingewogen (jeweils 2g) und anschließend 16ml Dimethylformamid als Lösungsmittel hinzugefügt. Der Ansatz wurde bei 20°C 24 Stunden auf einem Magnetrührer gerührt. Die Lösung wurde anschließend mit Hilfe eines 120µm-Rakels auf einer Glasplatte ausgezogen. Diese wurde 0,5 Stunden bei RT im Abzug getrocknet und anschließend im Trockenschrank bei 50°C und 10mbar für weitere 0,5 Stunden getrocknet, um das Lösungsmittel quantitativ zu entfernen. Die Proben wurden anschließend visuell begutachtet. Wenn die Filme klar waren und der Wirkstoff nach 7 Tagen nicht auskristallisierte, wurde der Wirkstoff als stabil im Polymer gelöst beurteilt (Angabe in Tabelle 1: 50). Wenn mit einem Wirkstoffanteil von 50 Gew.-% keine feste Lösung zu erzielen war, wurde der Versuch mit einer Wirkstoffbeladung von 33 Gew.-% wiederholt und die Bildung einer stabilen festen Lösung wie oben beurteilt (Angabe in Tabelle: 33).

**Stabilität einer festen Lösung**

| | | | | | |
|---|---|---|---|---|---|
| Feste Lösung Carbamazepin | Copolymer gemäß Beispiel Nr./ Wirkstoffgehalt [Gew.-%] | | | | |
| | 1 / 50 | 2/ 50 | 3/50 | 4/33 | 5/50 |

| | | | | | |
|---|---|---|---|---|---|
| Feste Lösung Estradiol | Copolymer gemäß Beispiel Nr./ Wirkstoffgehalt [Gew.-%] | | | | |
| | 1 / 50 | 2/ 50 | 3/50 | 4/50 | |

| | | | | | |
|---|---|---|---|---|---|
| Feste Lösung Piroxicam | Copolymer gemäß Beispiel Nr. / Wirkstoffgehalt [Gew.-%] | | | | |
| | 1 / 33 | 2/ 33 | 3/33 | 4/33 | 5/33 |

| | | | | | |
|---|---|---|---|---|---|
| Feste Lösung Clotrimazol | Copolymer gemäß Bsp.nr. / Solubilisierung bei 20 °C in [g/100 ml] | | | | |
| | 1/ 50 | 2/ 50 | 3/50 | 4/33 | 5/50 |

| | | | | | | | |
|---|---|---|---|---|---|---|---|
| Feste Lösung Carbamazepin | Copolymer gemäß Beispiel Nr./ Wirkstoffgehalt [Gew.-%] | | | | | | |
| | 6/ 50 | 7/ 50 | 8/50 | 12/ 33 | 13/ 50 | 14/ 50 | 15/ 33 |

| | | | | | | | | |
|---|---|---|---|---|---|---|---|---|
| Feste Lösung Estradiol | Copolymer gemäß Beispiel Nr./ Wirkstoffgehalt [Gew.-%] | | | | | | | |
| | 6/ 50 | 7/ 50 | 8/50 | 9/50 | 10/ 50 | 12/ 50 | 14/ 50 | 15/ 33 |

| | | | | | | | | | | | | | |
|---|---|---|---|---|---|---|---|---|---|---|---|---|---|
| Feste Lösung Piroxicam | Copolymer gemäß Beispiel Nr. / Wirkstoffgehalt [Gew.-%] | | | | | | | | | | | | |
| | 12/ 33 | | 13/ 33 | | 15/ 33 | | | | | | | | |
| Feste Lösung Clotrimazol | Copolymer gemäß Beispiel Nr./ Wirkstoffgehalt [Gew.-%] | | | | | | | | | | | | |
| | 6 50 | 7/ 50 | | 8/ 50 | | 9/ 50 | 10/ 50 | 11/ 33 | 12/ 50 | 13/ 33 | 14/ 50 | 15/ 33 | 16/ 50 |

| | | | | | | |
|---|---|---|---|---|---|---|
| Feste Lösung Carbamazepin | Copolymer gemäß Beispiel Nr./ Wirkstoffgehalt [Gew.-%] | | | | | |
| | 18/ 50 | 19/ 50 | 20/ 50 | 21/ 33 | 22/ 50 | 24/ 50 |

| | | | | | | |
|---|---|---|---|---|---|---|
| Feste Lösung Estradiol | Copolymer gemäß Beispiel Nr./ Wirkstoffgehalt [Gew.-%] | | | | | |
| | 18/ 50 | 19/ 50 | 20/ 50 | 21/ 50 | 22/ 50 | 24/ 50 |

| | | | | | | |
|---|---|---|---|---|---|---|
| Feste Lösung Piroxicam | Copolymer gemäß Beispiel Nr./ Wirkstoffgehalt [Gew.-%] | | | | | |
| | 18/ 33 | 19/ 33 | 20/ 33 | 21/ 33 | 22/ 33 | 24/ 33 |

| | | | | | | |
|---|---|---|---|---|---|---|
| Feste Lösung Clotrimazol | Copolymer gemäß Beispiel Nr./ Wirkstoffgehalt [Gew.-%] | | | | | |
| | 18/ 50 | 19/ 50 | 20/ 50 | 21/ 33 | 22/ 50 | 24/ 50 |

Mit den Polymerisaten gemäß den Vergleichsbeispielen A und B konnten keine stabilen festen Lösungen erhalten werden.

### Herstellung von Solubilisaten

In ein Becherglas wurden 2g des Copolymers eingewogen. Anschließend wurde dem Ansatz jeweils ein Arzneistoff wie folgt zugewogen, um eine übersättigte Lösung zu erhalten. Falls sich die eingewogene Masse im Medium auflöste, wurde die Einwaage bis zur Ausbildung eines Bodensatzes erhöht.
Zugewogene Menge an Wirkstoff: 17-β-Estradiol 0,2 g; Piroxicam 0,2 ; Clotrimazol 0,2 g; Carbamazepin 0,3 g; Ketoconazol 0,25 g; Griseofulvin 0,25 g; Cinnarizin 0,25 g.

Anschließend wurde Phosphatpuffer pH 7,0 hinzugegeben, bis Solubilisator und Phosphatpuffer im Gewichtsverhältnis von 1:10 vorlagen. Mit Hilfe eines Magnetrührers wurde dieser Ansatz bei 20°C 72 Stunden gerührt. Danach erfolgte mindestens eine 1 stündige Ruhezeit. Nach der Filtration des Ansatzes wurde dieser photometrisch vermessen und der Gehalt an Wirkstoff bestimmt.

| | | | | | |
|---|---|---|---|---|---|
| | Copolymer gemäß Bsp.nr. / Solubilisierung bei 20 °C in [g/100 ml] | | | | |
| Carbamazepin | 1/ 0,04 | 2/,07 | 3/0,40 | 4/0,28 | 5/0,26 |

| | | | | |
|---|---|---|---|---|
| | Copolymer gemäß Bsp.nr. / Solubilisierung bei 20 °C in [g/100 ml] | | | |
| Estradiol | 1/ 0,05 | 3/0,23 | 4/0,33 | 5/0,25 |

| | | | | | |
|---|---|---|---|---|---|
| Piroxicam | Copolymer gemäß Bsp.nr. / Solubilisierung bei 20 °C in [g/100 ml] | | | | |
| | 1 /0,11 | 2/ 0,18 | 3/0,28 | 4/0,09 | 5 /0,13 |

| | | | | |
|---|---|---|---|---|
| Clotrimazol | Copolymer gemäß Bsp.nr. / Solubilisierung bei 20 °C in [g/100 ml] | | | |
| | 1/ 0,01 | 2/ 0,01 | 3/ 0,17 | 4/ 0,15 |

| | | | | | | | | | | | |
|---|---|---|---|---|---|---|---|---|---|---|---|
| Carbamazepin | Copolymer gemäß Bsp.nr. / Solubilisierung bei 20 °C in [g/100 ml] | | | | | | | | | | |
| | 6/ 0,31 | 7/ 0,35 | 8/ 0,22 | 9/ 0,22 | 10/ 0,19 | 11/ 0,19 | 12/ 0,26 | 13/ 0,12 | 14/ 0,17 | A/ 0,10 | B/ 0,11 |

| | | | | | | | | |
|---|---|---|---|---|---|---|---|---|
| Estradiol | Copolymer gemäß Bsp.nr. / Solubilisierung bei 20 °C in [g/100 ml] | | | | | | | |
| | 6/0,23 | 7/ 0,18 | 8/ 0,17 | 9/0,09 | 10/ 0,09 | 14/ 0,04 | A/ 0,07 | B/0,11 |

| | | | | | | | | | | | | |
|---|---|---|---|---|---|---|---|---|---|---|---|---|
| Piroxicam | Copolymer gemäß Bsp.nr. / Solubilisierung bei 20 °C in [g/100 ml] | | | | | | | | | | | |
| | 6/ 0,45 | 7/ 0,45 | 8/ 0,44 | 9/ 0,42 | 10/ 0,44 | 11/ 0,39 | 12/ 0,45 | 14/ 0,42 | 16/ 0,37 | 17/ 0,4 | A/ 0,10 | B/ 0,27 |

| | | | | |
|---|---|---|---|---|
| Clotrimazol | Copolymer gemäß Bsp.nr. / Solubilisierung bei 20 °C in [g/100 ml] | | | |
| | 6/0,11 | 7/ 0,10 | 8/0,06 | 9/0,07 |

| | | | | |
|---|---|---|---|---|
| Carbamazepin | Copolymer gemäß Bsp.nr. / Solubilisierung bei 20 °C in [g/100 ml] | | | |
| | 18/ 0,31 | 19/ 0,25 | 20/ 0,28 | 21/ 0,27 |

| | | | | | | |
|---|---|---|---|---|---|---|
| Estradiol | Copolymer gemäß Bsp.nr. / Solubilisierung bei 20 °C in [g/100 ml] | | | | | |
| | 18/ 0,26 | 19/ 0,23 | 20/ 0,22 | 21/ 0,23 | 22/ 0,26 | 24/ 0,32 |

| | | | | | | |
|---|---|---|---|---|---|---|
| Piroxicam | Copolymer gemäß Bsp.nr. / Solubilisierung bei 20 °C in [g/100 ml] | | | | | |
| | 18/ 0,17 | 19/ 0,09 | 20/ 0,41 | 21/ 0,19 | 22/ 0,16 | 24/ 0,10 |

| | | | | | |
|---|---|---|---|---|---|
| Clotrimazol | Copolymer gemäß Bsp.nr. / Solubilisierung bei 20 °C in [g/100 ml] | | | | |
| | 18/ 0,06 | 19/ 0,13 | 20/ 0,18 | 21/ 0,12 | 22/0,13 |

| | | | |
|---|---|---|---|
| Copolymer gemäß Bsp.6 | Solubilisierung bei 20 °C in [g/100 ml] | | |
| | Ketoconazol 0,11 | Griseofulvin 0,31 | Cinnarizin 0,02 |

Analog zu der oben beschriebenen Methode wurde die Solubiliserung bei 37 ^C bestimmt.

| | | | |
|---|---|---|---|
| Carbamazepin | Copolymer gemäß Bsp.nr./ Solubilisierung bei 37 °C in [g/100 ml] | | |
| | 1/ 0,36 | 7/ 0,93 | 9/0,26 |
| Piroxicam | Copolymer gemäß Bsp.nr./ Solubilisierung bei 37 °C in [g/100 ml] | | |
| | 1/ 0,51 | 7/ 1,04 | 9/0,55 |
| Estradiol | Copolymer gemäß Bsp.nr./ Solubilisierung bei 37 °C in [g/100 ml] | | |
| | 1/ 0,99 | | 9/ 0,07 |

| | |
|---|---|
| Clotrimazol | Copolymer gemäß Bsp.nr./ Solubilisierung bei 37 °C in [g/100 ml] |
| | 1 / 0,17 |

Bestimmung der Glasübergangstemperaturen T_{g} als Maß für die klebefreie Verarbeitbarkeit:

| | | | |
|---|---|---|---|
| T_{g}[°C] | Copolymer gemäß Bsp. 1 | Copolymer gemäß Vergleichsbsp. A | Copolymer gemäß Vergleichsbsp. B |
| | + 54 | - 32 | - 40 |

Weiterhin wurde das Freisetzungsverhalten von erfindungsgemäßen festen Lösungen aus Solubilisatorpolymer und Wirkstoff (im Beispiel: Carbamazepin) -formuliert in einer Tablette - mit dem Freisetzungsverhalten einer Tablette verglichen, die nur Wirkstoff ohne Solubilisatorpolymer enthält. Dafür wurden folgende Tablettenzusammensetzungen verwendet:

**Formulierung A: Feste Lösung**

| Materialien | mg/ Tablette | [%] |
|---|---|---|
| Feste Lösung | 250 | 50 |
| Mikrokristalline Cellulose | 230 | 46 |
| Carboxymethylcellulose-Natrium, vernetzt | 15 | 3 |
| Hochdisperse Kieselsäure | 2.5 | 0.5 |
| Magnesiumstearat | 2.5 | 0.5 |
| Gesamt:I | 500 | 100 |

**Formulierung B: Wirkstoff ohne Polymer (CBZ)**

| Materialien | mg/ Tablette | [%]t |
|---|---|---|
| Carbamazepin | 75 | 15 |
| Lactose | 175 | 35 |
| Mikrokristalline Cellulose | 230 | 46 |
| Carboxymethylcellulose-Natrium, vernetzt | 15 | 3 |
| Hochdisperse Kieselsäure | 2.5 | 0.5 |
| Magnesiumstearat | 2.5 | 0.5 |
| Gesamt | 500 | 100 |

Die feste Lösung wurde in einem Mörser pulverisiert; die Teilchengröße lag im Bereich von 200 µm. Die weiteren Hilfsstoffe mit Ausnahme des Magnesiumstearats werden entsprechend den Angaben in der Tabelle zugegeben und in einem Turbulamischer 10 Minuten gemischt. Danach wird die in der Tabelle angegebene Masse Magnesiumstearat zugegeben und erneut 2 Minuten im Turbulamischer gemischt. Die Mischung wurde auf der Excenterpresse EK 0 mit einem 12 mm Stempel bei einer Presskraft von 15 kN tablettiert.

Die Freisetzung aus den Tabletten wurde in 0.08 M HCl (300 ml) bei 22°C bestimmt. Proben wurden durch einen 10µm-Filter filtriert und der Gehalt an Carbamazepin UVspektrometrisch bei 286 nm bestimmt.

| Zeit [min] | CBZ Freisetzung [%] | Feste Lösung Freisetzung [%] |
|---|---|---|
| 0 | 0 | 0 |
| 10 | | 96 |
| 20 | 43 | 99 |
| 40 | 48 | 100 |
| 60 | 50 | 102 |
| 90 | 50 | 99 |
| 120 | 50 | 100 |

## Patentansprüche

1. Verwendung von Copolymeren, erhalten durch radikalisch initiierte Polymerisation einer Mischung aus
i) 30 bis 80 Gew.-% N-Vinylcaprolactam,
ii) 10 bis 50 Gew.-% Vinylacetat, und
iii) 10 bis 50 Gew.-% eines Polyethylenglykols,
mit der Maßgabe, dass die Summe der Komponenten i), ii) und iii) gleich 100 Gew.-% ist,
als Solubilisatoren für in Wasser schwerlösliche biologisch aktive Substanzen, wobei "in Wasser schwerlöslich" bedeutet, dass für eine Lösung der Substanz in Wasser bei 20 °C mindestens 30 bis 100 g Wasser pro g Substanz benötigt werden

2. Verwendung nach Anspruch 1, wobei die Copolymere aus
i) 30 bis 70 Gew.-% N-Vinylcaprolactam,
ii) 15 bis 35 Gew.-% Vinylacetat, und
iii) 10 bis 35 Gew.-% eines Polyethylenglykols,
erhalten werden.

3. Verwendung nach einem der Ansprüche 1 oder 2, wobei die Copolymeren als Komponente iii) ein Polyethylenglykol mit einem Molekulargewicht von 1000 Dalton bis 10.000 Dalton enthalten.

4. Verwendung nach einem der Ansprüche 1 bis 3 wobei die Copolymeren einen K-Wert von 10 bis 60, gemessen in einer 1 gew.-%igen ethanolischen Lösung, aufweisen.

5. Verwendung nach einem der Ansprüche 1 bis 4 wobei die Copolymeren einen K-Wert von 15 bis 40 ,gemessen in einer 1 gew.-%igen ethanolischen Lösung, aufweisen.

6. Verwendung nach einem der Ansprüche 1 bis 5, zur Herstellung von pharmazeutischen Zubereitungen.

7. Verwendung nach einem der Ansprüche 1 bis 5 für kosmetische Zubereitungen.

8. Verwendung nach einem der Ansprüche 1 bis 5 für agrochemische Zubereitungen.

9. Verwendung nach einem der Ansprüche 1 bis 5 für Nahrungsergänzungsmittel oder dietätische Mittel.

10. Zubereitungen von in Wasser schwerlöslichen biologisch aktiven Substanzen, enthaltend als Solubilisatoren Copolymere, erhältlich durch radikalisch initiierte Polymerisation einer Mischung aus
i) 30 bis 80 Gew.-% N-Vinylcaprolactam,
ii) 10 bis 50 Gew.-% Vinylacetat, und
iii) 10 bis 50 Gew.-% eines Polyethylenglykols,
mit der Maßgabe, dass die Summe der Komponenten i), ii) und iii) bezogen auf das Copolymer gleich 100 Gew.-%, ist, wobei "in Wasser schwerlöslich" bedeutet, dass für eine Lösung der Substanz in Wasser bei 20 °C mindestens 30 bis 100 g Wasser pro g Substanz benötigt werden, und wobei unter "biologisch aktive Substanzen" pharmazeutische, kosmetische oder agrochemische Wirkstoffe oder Nahrungsergänzungsmittel oder diätetische Wirkstoffe verstanden werden.

11. Zubereitungen nach Anspruch 10, in denen die in Wasser schwerlösliche biologisch aktive Substanz in Form einer festen Lösung in den Copolymeren vorliegt.

12. Zubereitungen nach einem der Ansprüche 10 oder 11, enthaltend als in Wasser schwerlösliche biologisch aktive Substanz einen pharmazeutischen Wirkstoff.

13. Zubereitungen nach Anspruch 12, in Form oral applizierbarer Darreichungsformen.

14. Zubereitungen nach einem der Ansprüche 10 oder 11, enthaltend als in Wasser schwerlösliche biologisch aktive Substanz einen kosmetischen Wirkstoff.

15. Zubereitungen nach einem der Ansprüche 10 oder 11, enthaltend als in Wasser schwerlösliche biologisch aktive Substanz einen agrochemischen Wirkstoff.

16. Zubereitungen nach einem der Ansprüche 10 oder 11, enthaltend als in Wasser schwerlösliche biologisch aktive Substanz ein Nahrungsergänzungsmittel oder einen dietätischen Wirkstoff.

## Claims

1. The use of copolymers obtained by free-radical polymerization of a mixture of
i) 30 to 80% by weight of N-vinylcaprolactam,
ii) 10 to 50% by weight of vinyl acetate, and
iii)10 to 50% by weight of a polyethylene glycol,
with the proviso that the total of components i), ii) and iii) equals 100% by weight,
as solubilizers for slightly water-soluble bioactive substances, where "slightly water-soluble" means that at least 30 to 100g of water are required per g of substance for the substance to be dissolved in water at 20°C.

2. The use according to claim 1, where the copolymers are obtained from
i)30 to 70% by weight of N-vinylcaprolactam,
ii) 15 to 35% by weight of vinyl acetate, and
iii)10 to 35% by weight of a polyethylene glycol.

3. The use according to either of claims 1 and 2, where the copolymers comprise a polyethylene glycol having a molecular weight of from 1000 daltons to 10 000 daltons as component iii).

4. The use according to any of claims 1 to 3, where the copolymers have a K value from 10 to 60, measured in a 1% by weight ethanolic solution.

5. The use according to any of claims 1 to 4, where the copolymers have a K value of from 15 to 40, measured in a 1% by weight ethanolic solution.

6. The use according to any of claims 1 to 5 for producing pharmaceutical preparations.

7. The use according to any of claims 1 to 5 for cosmetic preparations.

8. The use according to any of claims 1 to 5 for agrochemical preparations.

9. The use according to any of claims 1 to 5 for dietary supplements or dietetic compositions.

10. Preparations of slightly water-soluble bioactive substances comprising as solubilizers copolymers obtainable by free-radical polymerization of a mixture of
i) 30 to 80% by weight of N-vinylcaprolactam,
ii) 10 to 50% by weight of vinyl acetate, and
iii)10 to 50% by weight of a polyethylene glycol,
with the proviso that the total of components i), ii) and iii), based on the copolymer, equals 100% by weight, where "slightly water-soluble" means that at least 30 to 100g of water are required per g of substance for the substance to be dissolved in water at 20°C, and where "bioactive substances" mean pharmaceutical, cosmetic or agrochemical active ingredients or dietary supplements or dietetic active substances.

11. Preparations according to claim 10, in which the slightly water-soluble bioactive substance is present in the form of a solid solution in the copolymers.

12. Preparations according to either of claims 10 and 11, comprising an active pharmaceutical ingredient as slightly water-soluble bioactive substance.

13. Preparations according to claim 12, in the form of dosage forms which can be administered orally.

14. Preparations according to either of claims 10 and 11, comprising a cosmetic active substance as slightly water-soluble bioactive substance.

15. Preparations according to either of claims 10 and 11, comprising an agrochemical active substance as slightly water-soluble bioactive substance.

16. Preparations according to either of claims 10 and 11, comprising as slightly water-soluble bioactive substance a dietary supplement or dietetic composition.

## Revendications

1. Utilisation de copolymères obtenus par polymérisation initiée par voie radicalaire d'un mélange constitué par
i) 30 à 80% en poids de N-vinylcaprolactame,
ii) 10 à 50% en poids d'acétate de vinyle et
iii) 10 à 50% en poids d'un polyéthylèneglycol,
à condition que la somme des composants i), ii) et iii) soit égale à 100% en poids, comme agents de solubilisation pour des substances biologiquement actives, peu solubles dans l'eau, l'expression "peu soluble dans l'eau" signifiant qu'il faut au moins 30 à 100 g d'eau par g de substance pour une solution de la substance dans l'eau à 20°C.

2. Utilisation selon la revendication 1, les copolymères étant obtenus à partir de
i) 30 à 70% en poids de N-vinylcaprolactame,
ii) 15 à 35% en poids d'acétate de vinyle et
iii) 10 à 35% en poids d'un polyéthylèneglycol.

3. Utilisation selon l'une quelconque des revendications 1 ou 2, les copolymères contenant, comme composant iii), un polyéthylèneglycol présentant un poids moléculaire de 1000 daltons à 10.000 daltons.

4. Utilisation selon l'une quelconque des revendications 1 à 3, les copolymères présentant une valeur K de 10 à 60, mesurée dans une solution éthanolique à 1% en poids.

5. Utilisation selon l'une quelconque des revendications 1 à 4, les copolymères présentant une valeur K de 15 à 40, mesurée dans une solution éthanolique à 1% en poids.

6. Utilisation selon l'une quelconque des revendications 1 à 5 pour la préparation de compositions pharmaceutiques.

7. Utilisation selon l'une quelconque des revendications 1 à 5 pour des compositions cosmétiques.

8. Utilisation selon l'une quelconque des revendications 1 à 5 pour des compositions agrochimiques.

9. Utilisation selon l'une quelconque des revendications 1 à 5 pour des compléments alimentaires ou des produits diététiques.

10. Compositions de substances biologiquement actives, peu solubles dans l'eau, contenant, comme agents de solubilisation, des copolymères pouvant être obtenus par polymérisation initiée par voie radicalaire d'un mélange constitué par
i) 30 à 80% en poids de N-vinylcaprolactame,
ii) 10 à 50% en poids d'acétate de vinyle et
iii) 10 à 50% en poids d'un polyéthylèneglycol,
à condition que la somme des composants i), ii) et iii), par rapport au copolymère, soit égale à 100% en poids, l'expression "peu soluble dans l'eau" signifiant qu'il faut au moins 30 à 100 g d'eau par g de substance pour une solution de la substance dans l'eau à 20°C et l'expression "substances biologiquement actives" désignant des substances actives pharmaceutiques, cosmétiques ou agrochimiques ou des compléments alimentaires ou des substances actives diététiques.

11. Compositions selon la revendication 10, dans lesquelles la substance biologiquement active, peu soluble dans l'eau se trouve sous forme d'une solution solide dans les copolymères.

12. Compositions selon l'une quelconque des revendications 10 ou 11, contenant, comme substance biologiquement active, peu soluble dans l'eau, une substance active pharmaceutique.

13. Compositions selon la revendication 12, sous forme de formes posologiques administrables par voie orale.

14. Compositions selon l'une quelconque des revendications 10 ou 11, contenant, comme substance biologiquement active, peu soluble dans l'eau, une substance cosmétique.

15. Compositions selon l'une quelconque des revendications 10 ou 11, contenant, comme substance biologiquement active, peu soluble dans l'eau, une substance agrochimique.

16. Compositions selon l'une quelconque des revendications 10 ou 11, contenant, comme substance biologiquement active, peu soluble dans l'eau, un complément alimentaire ou une substance active diététique.
